# EUROPEAN PATENT APPLICATION

(11) **EP 3 680 341 A1**
(43) Date of publication of application: **15.07.2020**
(21) Application number: 18850287.6
(22) Date of filing: 04.09.2018
(51) Int. Cl.: C12P 19/18, A61K 8/60, A61K 8/73, A61K 31/7034, A61K 31/716, A61P 17/00, A61P 43/00, A61Q 19/00, C08B 33/04

(54) **MIXTURE OF INOSITOL DERIVATIVES**

(30) Priority: 04.09.2017 JP 2017169774
(71) Applicant: Showa Denko K.K., Tokyo 105-8518 (JP)
(72) Inventor: FUJITA Ichiro, Tokyo 105-8518 (JP); YAMAKI Shinji, Tokyo 105-8518 (JP); NAKAGAMI Yuko, Tokyo 105-8518 (JP)
(74) Representative: Strehl Schübel-Hopf & Partner
(86) International application number: PCT/JP2018/032683
(87) International publication number: WO 2019/045113

(57) **Abstract**

A mixture of inositol derivatives in which a sugar is bonded to inositol includes an inositol derivative (A10) in which total sugars bonded to one inositol molecule is 10 or more in terms of monosaccharide units. In addition, a mixture of inositol derivatives in which a sugar is bonded to inositol includes 5 % by mass or more of an inositol derivative (A7), in which total sugars bonded to one inositol molecule is 7 or more in terms of monosaccharide units, with respect to a total amount of inositol derivatives (100 % by mass).

## Description

### [Technical Field]

The present invention relates to a mixture of inositol derivatives. The present invention further relates to a cell-activating agent including the mixture of inositol derivatives, a composition for cell activation, an external preparation for skin, and a cosmetic preparation.

Priority is claimed on Japanese Patent Application No. 2017-169774, filed September 4, 2017, the content of which is incorporated herein by reference.

### [Background Art]

It is known that an inositol derivative in which a sugar is bonded to inositol may be blended into an external preparation for skin as an active ingredient and causes various effects on the skin.

For example, Patent Literature 1 discloses that an external preparation for skin including an inositol derivative disclosed in Patent Literature 1 has an effect of imparting a sufficient moist sensation and smoothness to the skin, and keeping the skin healthy. Patent Literature 2 discloses that an external preparation for skin including an inositol derivative disclosed in Patent Literature 2 improves a moisture retention function of the skin, thereby enhancing a skin barrier function. Patent Literature 3 discloses that an external preparation for skin including an inositol derivative disclosed in Patent Literature 3 has effects of improving a physical barrier of the horny layer, improving a physiological moisturizing function of the epidermis, improving a moisture retention function of the skin, and the like. Patent Literature 4 discloses that an external preparation for skin including an inositol derivative disclosed in Patent Literature 4 has a cell-activating action, and an action of alleviating cell damage due to exposure to ultraviolet rays.

As a method for producing an inositol derivative in which glucose or an oligosaccharide having glucose as a structural unit is bonded to inositol, a method is known in which inositol and dextrin are reacted in the presence of cyclodextrin glucanotransferase (Patent Literature 5).

By this reaction, a mixture of inositol derivatives, in which the number of monosaccharide units of sugars bonded to inositol are different, is obtained. For example, Patent Literature 5 discloses in the example that a mixture of inositol derivatives, in which the number of monosaccharide units of sugars bonded to one inositol molecule is 1 to 6, can be obtained.

### [Citation List]

### [Patent Literature]

[Patent Literature 1]
   Japanese Patent No. 4624831
[Patent Literature 2]
   PCT International Publication No. WO2016/067688
[Patent Literature 3]
   PCT International Publication No. WO2016/076310
[Patent Literature 4]
   Japanese Unexamined Patent Application, First Publication No. 2007-84484
[Patent Literature 5]
   Japanese Unexamined Patent Application, First Publication No. S63-196596

### [Summary of Invention]

### [Technical Problem]

However, none of Patent Literature 1 to Patent Literature 5 shows a composition of a mixture of inositol derivatives which has a high level of cell-activating effect. Accordingly, an object of the present invention is to provide a mixture of inositol derivatives which has an improved cell-activating effect, and use applications thereof.

### [Solution to Problem]

The present invention includes the following aspects.
(1) A mixture of inositol derivatives in which a sugar is bonded to inositol, the mixture of inositol derivatives including an inositol derivative (A10) in which total sugars bonded to one inositol molecule is 10 or more in terms of monosaccharide units.
(2) The mixture of inositol derivatives according to (1), including 1 % by mass or more of the inositol derivative (A10) with respect to a total amount of inositol derivatives (100 % by mass).
(3) The mixture of inositol derivatives according to (2), including 5 % by mass or more of the inositol derivative (A10) with respect to a total amount of inositol derivatives (100 % by mass).
(4) The mixture of inositol derivatives according to any one of (1) to (3), including an inositol derivative in which total sugars bonded to one inositol molecule is 11 or more in terms of monosaccharide units.
(5) The mixture of inositol derivatives according to (4), including an inositol derivative in which total sugars bonded to one inositol molecule is 12 or more in terms of monosaccharide units.
(6) The mixture of inositol derivatives according to any one of (1) to (5), in which the inositol derivative (A10) is at least one kind of inositol derivative selected from the group consisting of the following (a) and (b):
   (a) an inositol derivative in which one or more glucoses, and one or more oligosaccharides containing glucose as a structural unit are respectively bonded to inositol; and
   (b) an inositol derivative in which one or more oligosaccharides containing glucose as a structural unit are bonded to inositol.
(7) The mixture of inositol derivatives according to any one of (1) to (6), further including:
   an inositol derivative (B10) in which total sugars bonded to one inositol molecule is less than 10 in terms of monosaccharide units,
   wherein the inositol derivative (B10) is at least one kind of inositol derivatives selected from the group consisting of the following (a) to (c):
      (a) an inositol derivative in which one or more glucoses, and one or more oligosaccharides containing glucose as a structural unit are respectively bonded to inositol;
      (b) an inositol derivative in which one or more oligosaccharides containing glucose as a structural unit are bonded to inositol; and
      (c) an inositol derivative in which one or more glucoses are bonded to inositol.
(8) A mixture of inositol derivatives in which a sugar is bonded to inositol, the mixture of inositol derivatives including 5 % by mass or more of an inositol derivative (A7), in which total sugars bonded to one inositol molecule is 7 or more in terms of monosaccharide units, with respect to a total amount of inositol derivatives (100 % by mass).
(9) The mixture of inositol derivatives according to (8), in which the inositol derivative (A7) is at least one kind of inositol derivatives selected from the group consisting of the following (a) and (b):
   (a) an inositol derivative in which one or more glucoses, and one or more oligosaccharides containing glucose as a structural unit are respectively bonded to inositol; and
   (b) an inositol derivative in which one or more oligosaccharides containing glucose as a structural unit are bonded to inositol.
(10) The mixture of inositol derivatives according to (8) or (9), further including:
   an inositol derivative (B7) in which total sugars bonded to one inositol molecule is less than 7 in terms of monosaccharide units,
   wherein the inositol derivative (B7) is at least one kind of inositol derivatives selected from the group consisting of the following (a) to (c):
      (a) an inositol derivative in which one or more glucoses, and one or more oligosaccharides containing glucose as a structural unit are respectively bonded to inositol;
      (b) an inositol derivative in which one or more oligosaccharides containing glucose as a structural unit are bonded to inositol; and
      (c) an inositol derivative in which one or more glucoses are bonded to inositol.
(11) The mixture of inositol derivatives according to any one of (1) to (10), in which the inositol is myo-inositol.
(12) The mixture of inositol derivatives according to any one of (1) to (11), which promotes cell activation.
(13) A cell-activating agent, including the mixture of inositol derivatives according to any one of (1) to (12).
(14) A composition for cell activation, including the mixture of inositol derivatives according to any one of (1) to (12).
(15) An external preparation for skin, including the mixture of inositol derivatives according to any one of (1) to (12).
(16) A cosmetic preparation, including the mixture of inositol derivatives according to any one of (1) to (12).

### [Advantageous Effects of Invention]

According to the present invention, a mixture of inositol derivatives which has an improved cell-activating effect, and use applications thereof are provided.

### [Description of Embodiments]

### [Mixture of inositol derivatives]

In one embodiment, the present invention provides a mixture of inositol derivatives in which a sugar is bonded to inositol. The mixture of inositol derivatives of the present embodiment is a mixture of inositol derivatives of a first embodiment or a second embodiment to be described later.

### (Inositol derivatives)

Inositol is a cyclic hexahydric alcohol represented by C₆H₆(OH)₆. There are nine stereoisomeric forms of inositol: cis-inositol, epi-inositol, allo-inositol, myo-inositol, muco-inositol, neo-inositol, chiro-inositol (D-chiro- and L-chiro-), and scyllo-inositol.

The inositol constituting an inositol derivative is preferably myo-inositol which is physiologically active among the above-mentioned isomeric forms. Inositol can be synthesized by a method of extraction from rice bran, a chemical synthesis method, a fermentation method, or the like. A structural formula of myo-inositol is shown below.

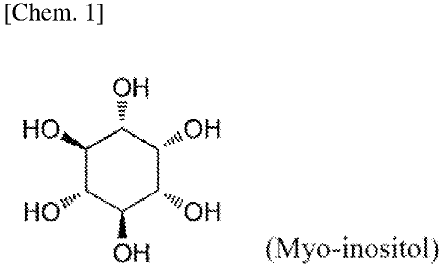

An inositol derivative is a compound in which a sugar is bonded to a hydroxyl group of inositol. The sugar may be bonded to any one of six hydroxyl groups present in an inositol molecule, or may be bonded to any two or more thereof.

A sugar that is bonded to inositol may be a monosaccharide or an oligosaccharide. For example, one or more monosaccharides may be bonded to one inositol molecule, one or more oligosaccharides may be bonded to one inositol molecule, or one or more monosaccharides and one or more oligosaccharides may be bonded to one inositol molecule. In the inositol derivatives, total sugars (monosaccharides and/or oligosaccharides) bonded to one inositol molecule in terms of monosaccharide units is 1 or more, may be 2 or more for example, may be 3 or more for example, and may be 4 or more for example.

In the present specification, a monosaccharide refers to a sugar group that cannot be hydrolyzed further, and refers to a compound that is a constituent element when forming a polysaccharide. A monosaccharide can also be said to be the smallest structural unit of a sugar group. In the present specification, the term "monosaccharide unit" refers to a chemical structure corresponding to a monosaccharide. A "monosaccharide unit" can also be said to be a chemical structure derived from a monosaccharide. For example, a disaccharide is converted into two monosaccharide units, and a trisaccharide is converted into three monosaccharide units. More specifically, for example, mannitol, sorbitol, xylitol, erythritol, pentaerythritol, glucose, fructose, xylose, or the like is converted into one monosaccharide unit.

In addition, maltitol, sucrose, lactose, maltose, trehalose, or the like is converted into two monosaccharide units. Furthermore, for example,α-cyclodextrin is converted into six monosaccharide units;β-cyclodextrin is converted into seven monosaccharide units; and γ-cyclodextrin is converted into eight monosaccharide units.

A sugar constituting the inositol derivatives is not particularly limited, and examples thereof include mannitol, sorbitol, xylitol, maltitol, erythritol, pentaerythritol, glucose, sucrose, fructose, lactose, maltose, xylose, trehalose, dextrin, α-cyclodextrin, β-cyclodextrin, γ-cyclodextrin, and the like.

The sugar constituting the inositol derivatives may be glucose, or an oligosaccharide containing glucose as a structural unit. The oligosaccharide may contain only glucose as a structural unit. Alternatively, the oligosaccharide may contain at least one glucose molecule, and a sugar other than glucose as structural units. A molecular weight of the above-mentioned oligosaccharide may be, for example, about 300 to 3000. More specific examples of oligosaccharides include disaccharides such as sucrose, lactose, maltose, trehalose, and cellobiose; trisaccharides such as raffinose, melezitose, and maltotriose; tetrasaccharides such as stachyose; and the like.

From the viewpoint of easily obtaining highly purified inositol derivatives, it is preferable to use β-cyclodextrin which is industrially inexpensive and can be stably supplied as a raw material for inositol derivatives. In this case, the sugar constituting the inositol derivatives contains glucose as a structural unit. Meanwhile, when cheaper starch or the like is used as a raw material for inositol derivatives, various sugars are transferred to various places during synthesis of inositol derivatives, and thus the degree of purification of inositol derivatives to be obtained tends to become unstable.

In addition, the inositol derivative may be in a form of a pharmaceutically acceptable salt. In the present specification, "pharmaceutically acceptable salt" refers to a salt form that does not inhibit a cell-activating effect of the inositol derivative. The pharmaceutically acceptable salts of the inositol derivative are not particularly limited, and examples thereof include salts with alkali metals (sodium, potassium, and the like); salts with alkaline earth metals (magnesium, calcium, and the like); salts with organic bases (pyridine, triethylamine, and the like); salts with amines; salts with organic acids (acetic acid, formic acid, propionic acid, fumaric acid, maleic acid, succinic acid, tartaric acid, citric acid, malic acid, oxalic acid, benzoic acid, methanesulfonic acid, and the like); salts with inorganic acids (hydrochloric acid, phosphoric acid, hydrobromic acid, sulfuric acid, nitric acid, and the like); and the like.

In addition, the inositol derivative may be in a form of a solvate. Furthermore, the inositol derivative may be in a form of a solvate of a salt of the inositol derivative. The solvate is not particularly limited, but examples thereof include hydrates, ethanol solvates, and the like.

### (Mixture of inositol derivatives)

The mixture of inositol derivatives includes inositol derivatives of two or more kinds. The inositol derivatives of two or more kinds are different from each other in a composition of sugar bonded to one inositol molecule. For example, the total numbers of sugar bonded to one inositol molecule in terms of monosaccharide units may be different from each other, and the types of sugar bonded to one inositol molecule may be different from each other. The number of kinds of inositol derivatives included in the mixture of inositol derivatives is not particularly limited, and it may be about 2 to 100 kinds. Examples of the mixture of inositol derivatives include a mixture of inositol derivatives which has about 2 to 30 kinds of sugar bonded to one inositol molecule in terms of the number of monosaccharide units. The mixture of inositol derivatives preferably has 5 or more kinds, more preferably has 10 or more kinds, even more preferably has 11 or more kinds, and particularly more preferably has 12 or more kinds of sugar bonded to one inositol molecule in terms of the number of monosaccharide units.

The mixture of inositol derivatives may include components other than the inositol derivatives (hereinafter referred to as "impurities"). Examples of impurities include raw materials, enzymes, catalysts, solvents, and the like which are used in the production of the mixture of inositol derivatives; intermediate products and by-products which are generated in the production process of the mixture of inositol derivatives; and the like. Examples of proportions of the impurities in the mixture of inositol derivatives include 10 % by mass or less. The proportion of impurities in the mixture of inositol derivatives is preferably 7 % by mass or less, is more preferably 5 % by mass or less, is even more preferably 3 % by mass or less, and is particularly preferably 1 % by mass or less.

### <First embodiment>

The mixture of inositol derivatives of the present embodiment is characterized by including an inositol derivative (A10) in which total sugars bonded to one inositol molecule is 10 or more in terms of monosaccharide units.

In the present specification, an "inositol derivative in which total sugars bonded to one inositol molecule is 10 or more in terms of monosaccharide units" may be expressed as an "inositol derivative having 10 or more monosaccharide units." Other inositol derivatives may be described in the same manner. Alternatively, an "inositol derivative in which total sugars bonded to one inositol molecule is 10 or more in terms of monosaccharide units" may be expressed as the "inositol derivative (A10)." Other inositol derivatives may be described in the same manner. For example, an "inositol derivative in which total sugars bonded to one inositol molecule is 7 or more in terms of monosaccharide units" may be expressed as the "inositol derivative (A7)." Meanwhile, an "inositol derivative in which total sugars bonded to one inositol molecule is less than 10 in terms of monosaccharide units" may be expressed as the "inositol derivative (B10)." Similarly, for other inositol derivatives, for example, an "inositol derivative in which total sugars bonded to one inositol molecule is less than 7 in terms of monosaccharide units" may be expressed as the "inositol derivative (B7)."

### (Inositol derivative having 10 or more monosaccharide units: inositol derivative (A10))

As will be described later in the Examples, the mixture of inositol derivatives of the present embodiment includes an inositol derivative having 10 or more monosaccharide units, thereby improving a cell-activating effect. In the present specification, the term "cell activation" means that a cell proliferative activity is improved. That is, the "cell-activating effect" means an action of improving a cell proliferative activity.

As long as the inositol derivative having 10 or more monosaccharide units has 10 or more sugars in terms of monosaccharide units, other configurations (a position and the number of hydroxyl groups to which a sugar is bonded, types of sugars, and the like) are not particularly limited. The inositol derivative having 10 or more monosaccharide units may be, for example, an inositol derivative in which one oligosaccharide molecule having 10 or more monosaccharide units is bonded to any one of 6 hydroxyl groups in an inositol molecule. In addition, for example, it may be an inositol derivative in which a monosaccharide and an oligosaccharide which have a total of 10 or more monosaccharide units are bonded to any two or more of 6 hydroxyl groups in an inositol molecule. In addition, for example, it may be an inositol derivative in which oligosaccharides having a total of 10 or more monosaccharide units are bonded to any two or more of 6 hydroxyl groups in an inositol molecule. In a case where sugars are bonded to two or more hydroxyl groups in an inositol molecule, the sugars bonded to each hydroxyl group may be the same as or different from each other.

In one embodiment, a sugar included in the inositol derivative having 10 or more monosaccharide units is glucose or an oligosaccharide having glucose as a structural unit. Examples of such inositol derivatives include at least one kind of inositol derivatives selected from the group consisting of the following (a) and (b):
(a) an inositol derivative in which one or more glucoses, and one or more oligosaccharides containing glucose as a structural unit are respectively bonded to inositol;
(b) an inositol derivative in which one or more oligosaccharides containing glucose as a structural unit are bonded to inositol.

In (a) and (b), the oligosaccharide containing glucose as a structural unit is preferably an oligosaccharide containing only glucose as a structural unit.

The number of monosaccharide units of sugars included in an inositol derivative having 10 or more monosaccharide units is not particularly limited as long as it is 10 or more. The number of monosaccharide units of sugars included in an inositol derivative having 10 or more monosaccharide units, for example, may be 10 to 50, preferably 10 to 30, more preferably 10 to 20, and even more preferably 10 to 15. In addition, the number of monosaccharide units of sugars included in the inositol derivative having 10 or more monosaccharide units may be 11 or more. In this case, the number of monosaccharide units of sugars is, for example, 11 to 50, preferably 11 to 30, more preferably 11 to 20, and even more preferably 11 to 15. In addition, the number of monosaccharide units of sugars included in the inositol derivative having 10 or more monosaccharide units may be 12 or more. In this case, the number of monosaccharide units of sugars is, for example, 12 to 50, preferably 12 to 30, more preferably 12 to 20, and even more preferably 12 to 15.

In addition, the mixture of inositol derivatives of the present embodiment may include two or more kinds of inositol derivatives having 10 or more monosaccharide units. For example, the mixture of inositol derivatives of the present embodiment may include an inositol derivative having 10 monosaccharide units and an inositol derivative having 11 or more monosaccharide units. In addition, for example, it may include an inositol derivative having 10 monosaccharide units and an inositol derivative having 12 or more monosaccharide units. Furthermore, for example, it may include an inositol derivative having 11 monosaccharide units and an inositol derivative having 12 or more monosaccharide units. Furthermore, for example, it may include an inositol derivative having 10 monosaccharide units, an inositol derivative having 11 monosaccharide units, and an inositol derivative having 12 or more monosaccharide units. In a preferable aspect, the mixture of inositol derivatives of the present embodiment includes an inositol derivative having 10 monosaccharide units, an inositol derivative having 11 monosaccharide units, and an inositol derivative having 12 or more monosaccharide units.

In the mixture of inositol derivatives of the present embodiment, a proportion of inositol derivative having 10 or more monosaccharide units is, for example, 5 % by mass or more with respect to a total amount (100 % by mass) of the inositol derivatives. A proportion of inositol derivative having 10 or more monosaccharide units is preferably 1 to 50 % by mass, more preferably 3 to 50 % by mass, even more preferably 5 to 50 % by mass, still more preferably 10 to 30 % by mass, and more preferably 10 to 20 % by mass with respect to a total amount (100 % by mass) of the inositol derivatives. In a case where a proportion of inositol derivative having 10 or more monosaccharide units is within the above-mentioned ranges, a higher level of cell-activating effect can be obtained. In the case where the mixture of inositol derivatives includes two or more kinds of inositol derivatives having 10 or more monosaccharide units, a total proportion thereof is preferably within the above-mentioned ranges. A total proportion of inositol derivatives having 10 or more monosaccharide units may be 100 % by mass.

In a case where the mixture of inositol derivatives of the present embodiment includes an inositol derivative having 11 or more monosaccharide units, a proportion thereof is, for example, 5 % by mass or more with respect to a total amount (100 % by mass) of the inositol derivatives. A proportion of inositol derivative having 11 or more monosaccharide units is preferably 1 to 50 % by mass, more preferably 3 to 50 % by mass, even more preferably 5 to 50 % by mass, still more preferably 10 to 30 % by mass, and more preferably 10 to 20 % by mass with respect to a total amount (100 % by mass) of the inositol derivatives. In a case where the mixture of inositol derivatives of the present embodiment includes an inositol derivative having 12 or more monosaccharide units, a proportion thereof is, for example, 3 % by mass or more with respect to a total amount (100 % by mass) of the inositol derivatives. A proportion of inositol derivative having 12 or more monosaccharide units is preferably 1 to 40 % by mass, preferably 3 to 40 % by mass, more preferably 5 to 25 % by mass, and even more preferably 6 to 15 % by mass with respect to a total amount (100 % by mass) of the inositol derivatives.

In the case where the mixture of inositol derivatives of the present embodiment includes two or more kinds of inositol derivatives having 10 or more monosaccharide units, a ratio thereof is not particularly limited. For example, in a case where the mixture of inositol derivatives of the present embodiment includes an inositol derivative having 10 monosaccharide units and an inositol derivative having 11 or more monosaccharide units, a mass ratio between the inositol derivative having 10 monosaccharide units and the inositol derivative having 11 or more monosaccharide units may be 1:0.5 to 1:10, may be 1:1 to 1:10, may be 1:1.5 to 1:5, or may be 1:2 to 1:4. For example, in a case where the mixture of inositol derivatives of the present embodiment includes an inositol derivative having 10 monosaccharide units and an inositol derivative having 12 or more monosaccharide units, a mass ratio between the inositol derivative having 10 monosaccharide units and the inositol derivative having 12 or more monosaccharide units may be 1:0.5 to 1:10, may be 1:1 to 1:10, may be 1:1 to 1:5, or may be 1:1 to 1:3. For example, in a case where the mixture of inositol derivatives of the present embodiment includes an inositol derivative having 11 monosaccharide units and an inositol derivative having 12 or more monosaccharide units, a mass ratio between the inositol derivative having 11 monosaccharide units and the inositol derivative having 12 or more monosaccharide units may be 1:0.5 to 1:10, may be 1:1 to 1:10, may be 1:1 to 1:5, or may be 1:1 to 1:3. For example, in a case where the mixture of inositol derivatives of the present embodiment includes an inositol derivative having 10 monosaccharide units, an inositol derivative having 11 monosaccharide units, and an inositol derivative having 12 or more monosaccharide units, a mass ratio between the inositol derivative having 10 monosaccharide units and the inositol derivative having 11 monosaccharide units may be 1:0.1 to 1:10, may be 1:0.5 to 1:5, or may be 1:0.7 to 1:2, and a mass ratio between the inositol derivative having 10 monosaccharide units and the inositol derivative having 12 or more monosaccharide units may be 1:0.5 to 1:10, may be 1:1 to 1:10, may be 1:1 to 1:5, or may be 1:1 to 1:3.

### (Inositol derivative having less than 10 monosaccharide units: inositol derivative (B10))

The mixture of inositol derivatives of the present embodiment may include, in addition to an inositol derivative having 10 or more monosaccharide units, an inositol derivative having less than 10 monosaccharide units. In a case where the mixture of inositol derivatives of the present embodiment includes only one kind of inositol derivative having 10 or more monosaccharide units, the mixture of inositol derivatives of the present embodiment includes at least one kind of inositol derivative having less than 10 monosaccharide units.

As in the case of the inositol derivatives having 10 or more monosaccharide units, a configuration (a position and the number of hydroxyl groups to which a sugar is bonded, types of sugar, and the like) of the inositol derivative having less than 10 monosaccharide units which can be included in the mixture of inositol derivatives of the present embodiment is not particularly limited except for the number of monosaccharide units of sugars bonded to one inositol molecule.

In one embodiment, a sugar included in the inositol derivative having less than 10 monosaccharide units is glucose or an oligosaccharide having glucose as a structural unit. Examples of such inositol derivatives include at least one kind of inositol derivatives selected from the group consisting of the following (a) to (c):
(a) an inositol derivative in which one or more glucoses, and one or more oligosaccharides containing glucose as a structural unit are respectively bonded to inositol;
(b) an inositol derivative in which one or more oligosaccharides containing glucose as a structural unit are bonded to inositol; and
(c) an inositol derivative in which one or more glucoses are bonded to inositol.

In (a) and (b), the oligosaccharide containing glucose as a structural unit is preferably an oligosaccharide containing only glucose as a structural unit.

Examples of inositol derivatives having less than 10 monosaccharide units include inositol derivatives having 1 to 9 monosaccharide units. The mixture of inositol derivatives of the present embodiment may include all of the inositol derivatives having 1 to 9 monosaccharide units, or may include any one or two or more kinds of each of the inositol derivatives having 1 to 9 monosaccharide units. In a preferable aspect, the mixture of inositol derivatives of the present embodiment includes all of the inositol derivatives having 1 to 9 monosaccharide units.

In one aspect, the mixture of inositol derivatives of the present embodiment may include 5 % by mass or more of an inositol derivative having 7 or more monosaccharide units.

Specific examples of the mixture of inositol derivatives of the present embodiment include a mixture having a composition shown in Table 1, for example. In Table 1, the term "% by mass" refers to a proportion with respect to a total amount (100 % by mass) of inositol derivatives contained in the mixture of inositol derivatives.

**[Table 1]**

| Type of inositol derivative | % by mass |
|---|---|
| Inositol derivative having one monosaccharide unit | 0 to 15 |
| Inositol derivative having two monosaccharide units | 0 to 25 |
| Inositol derivative having three monosaccharide units | 0 to 25 |
| Inositol derivative having four monosaccharide units | 0 to 25 |
| Inositol derivative having five monosaccharide units | 0 to 25 |
| Inositol derivative having six monosaccharide units | 0 to 25 |
| Inositol derivative having seven monosaccharide units | 0 to 25 |
| Inositol derivative having eight monosaccharide units | 0 to 15 |
| Inositol derivative having nine monosaccharide units | 0 to 15 |
| Inositol derivative having ten monosaccharide units | 0 to 10 |
| Inositol derivative having eleven monosaccharide units | 0 to 10 |
| Inositol derivative having twelve or more monosaccharide units | 1 to 20 |

Preferred specific examples of the mixture of inositol derivatives of the present embodiment include a mixture having a composition shown in Table 2, for example. The term "% by mass" in Table 2 is the same as that in Table 1.

**[Table 2]**

| Type of inositol derivative | % by mass |
|---|---|
| Inositol derivative having one monosaccharide unit | 4 to 14 |
| Inositol derivative having two monosaccharide units | 7 to 17 |
| Inositol derivative having three monosaccharide units | 7 to 17 |
| Inositol derivative having four monosaccharide units | 7 to 17 |
| Inositol derivative having five monosaccharide units | 6 to 16 |
| Inositol derivative having six monosaccharide units | 4 to 14 |
| Inositol derivative having seven monosaccharide units | 3 to 13 |
| Inositol derivative having eight monosaccharide units | 1 to 11 |
| Inositol derivative having nine monosaccharide units | 0 to 10 |
| Inositol derivative having ten monosaccharide units | 1 to 10 |
| Inositol derivative having eleven monosaccharide units | 1 to 10 |
| Inositol derivative having twelve or more monosaccharide units | 3 to 15 |

### <Second embodiment>

The mixture of inositol derivatives of the present embodiment is characterized by including 5 % by mass or more of an inositol derivative (A7), in which total sugars bonded to one inositol molecule is 7 or more in terms of monosaccharide units, with respect to a total amount of inositol derivatives (100 % by mass).

### (Inositol derivative having 7 or more monosaccharide units: inositol derivative (A7))

As will be described later in the Examples, the mixture of inositol derivatives of the present embodiment includes 5 % by mass or more of an inositol derivative having 7 or more monosaccharide units with respect to a total amount (100 % by mass) of inositol derivatives, thereby improving a cell-activating effect.

As long as the inositol derivative having 7 or more monosaccharide units has 7 or more sugars in terms of monosaccharide units, other configurations (a position and the number of hydroxyl groups to which a sugar is bonded, types of sugars, and the like) are not particularly limited. The inositol derivative having 7 or more monosaccharide units may be, for example, an inositol derivative in which one oligosaccharide molecule having 7 or more monosaccharide units is bonded to any one of 6 hydroxyl groups in an inositol molecule. In addition, for example, it may be an inositol derivative in which a monosaccharide and an oligosaccharide which have a total of 7 or more monosaccharide units are bonded to any two or more of 6 hydroxyl groups in an inositol molecule. In addition, for example, it may be an inositol derivative in which oligosaccharides having a total of 7 or more monosaccharide units are bonded to any two or more of 6 hydroxyl groups in an inositol molecule. In a case where sugars are bonded to two or more hydroxyl groups in an inositol molecule, sugars bonded to each hydroxyl group may be the same as or different from each other.

In one embodiment, a sugar included in the inositol derivative having 7 or more monosaccharide units is glucose or an oligosaccharide having glucose as a structural unit. Examples of such inositol derivatives include at least one kind of inositol derivatives selected from the group consisting of the following (a) and (b):
(a) an inositol derivative in which one or more glucoses, and one or more oligosaccharides containing glucose as a structural unit are respectively bonded to inositol; and
(b) an inositol derivative in which one or more oligosaccharides containing glucose as a structural unit are bonded to inositol.

In (a) and (b), the oligosaccharide containing glucose as a structural unit is preferably an oligosaccharide containing only glucose as a structural unit.

The number of monosaccharide units of sugars included in the inositol derivative having 7 or more monosaccharide units is not particularly limited as long as it is 7 or more. The number of monosaccharide units of sugars included in the inositol derivative having 7 or more monosaccharide units is, for example, 7 to 50, preferably 7 to 30, more preferably 7 to 20, and even more preferably 7 to 15.

In one aspect, the mixture of inositol derivatives of the present embodiment may be a mixture of inositol derivatives which includes 5 % by mass or more of an inositol derivative having 7 or more monosaccharide units with respect to a total amount (100 % by mass) of inositol derivatives, and includes an inositol derivative having 10 or more monosaccharide units.

In addition, the mixture of inositol derivatives of the present embodiment may be a mixture of inositol derivatives which includes 5 % by mass or more of an inositol derivative having 7 or more monosaccharide units with respect to a total amount (100 % by mass) of inositol derivatives, and includes an inositol derivative having 11 or more monosaccharide units. The mixture of inositol derivatives of the present embodiment may be a mixture of inositol derivatives which includes 5 % by mass or more of an inositol derivative having 7 or more monosaccharide units with respect to a total amount (100 % by mass) of inositol derivatives, and includes an inositol derivative having 12 or more monosaccharide units.

The mixture of inositol derivatives of the present embodiment may include two or more kinds of inositol derivatives having 7 or more monosaccharide units. For example, the mixture of inositol derivatives of the present embodiment may include inositol derivatives having 7, 8, 9, 10, and 11 monosaccharide units, and an inositol derivative having 12 or more monosaccharide units, or may include any one or two or more kinds of these inositol derivatives. In a preferable aspect, the mixture of inositol derivatives of the present embodiment includes inositol derivatives having 7, 8, 9, 10, and 11 monosaccharide units, and an inositol derivative having 12 or more monosaccharide units.

In the mixture of inositol derivatives of the present embodiment, a proportion of inositol derivative having 7 or more monosaccharide units is not particularly limited as long as it is 5 % by mass or more with respect to a total amount (100 % by mass) of the inositol derivatives. A proportion of inositol derivative having 7 or more monosaccharide units is, for example, 5 to 99 % by mass with respect to a total amount (100 % by mass) of the inositol derivatives.

A proportion of inositol derivative having 7 or more monosaccharide units is preferably 10 to 80 % by mass, more preferably 15 to 60 % by mass, even more preferably 20 to 50 % by mass, still even more preferably 25 to 45 % by mass, and particularly preferably 30 to 40 % by mass with respect to a total amount (100 % by mass) of the inositol derivatives. In a case where a proportion of inositol derivative having 7 or more monosaccharide units is within the above-mentioned ranges, a higher level of cell-activating effect can be obtained. In the case where the mixture of inositol derivatives includes two or more kinds of inositol derivatives having 7 or more monosaccharide units, a total proportion thereof is preferably within the above-mentioned ranges. A total proportion of inositol derivatives having 7 or more monosaccharide units may be 100 % by mass.

In the case where the mixture of inositol derivatives of the present embodiment includes two or more kinds of inositol derivatives having 7 or more monosaccharide units, a proportion thereof is not particularly limited. For example, in a case where the mixture of inositol derivatives of the present embodiment includes an inositol derivative having 7 monosaccharide units and an inositol derivative having 8 or more monosaccharide units, a mass ratio between the inositol derivative having 7 monosaccharide units and the inositol derivative having 8 or more monosaccharide units may be 1:1 to 1:10, may be 1:1 to 1:5, or may be 1:2 to 1:4.

### (Inositol derivative having less than 7 monosaccharide units: inositol derivative (B7))

The mixture of inositol derivatives of the present embodiment may include, in addition to an inositol derivative having 7 or more monosaccharide units, an inositol derivative having less than 7 monosaccharide units. In a case where the mixture of inositol derivatives of the present embodiment includes only one kind of inositol derivative having 7 or more monosaccharide units, the mixture of inositol derivatives of the present embodiment includes at least one kind of inositol derivative having less than 7 monosaccharide units.

As in the case of the inositol derivatives having 7 or more monosaccharide units, a configuration (a position and the number of hydroxyl groups to which a sugar is bonded, types of sugar, and the like) of the inositol derivative having less than 7 monosaccharide units which can be included in the mixture of inositol derivatives of the present embodiment is not particularly limited except for the number of monosaccharide units of sugars bonded to one inositol molecule.

In one embodiment, a sugar included in the inositol derivative having less than 7 monosaccharide units is glucose or an oligosaccharide having glucose as a structural unit. Examples of such inositol derivatives include at least one kind of inositol derivatives selected from the group consisting of the following (a) to (c):
(a) an inositol derivative in which one or more glucoses, and one or more oligosaccharides containing glucose as a structural unit are respectively bonded to inositol;
(b) an inositol derivative in which one or more oligosaccharides containing glucose as a structural unit are bonded to inositol; and
(c) an inositol derivative in which one or more glucoses are bonded to inositol.

In (a) and (b), the oligosaccharide containing glucose as a structural unit is preferably an oligosaccharide containing only glucose as a structural unit.

Examples of inositol derivatives having less than 7 monosaccharide units include inositol derivatives having 1 to 6 monosaccharide units. The mixture of inositol derivatives of the present embodiment may include all of the inositol derivatives having 1 to 6 monosaccharide units, or may include any one or two or more kinds of each of the inositol derivatives having 1 to 6 monosaccharide units. In a preferable aspect, the mixture of inositol derivatives of the present embodiment includes all of the inositol derivatives having 1 to 6 monosaccharide units.

The mixtures of the inositol derivatives of the first embodiment and the second embodiment have an excellent cell-activating effect. Accordingly, in one aspect, the present invention also provides a mixture of inositol derivatives of the first embodiment or the second embodiment which promotes cell activation.

Since the mixtures of the inositol derivatives of the first embodiment and the second embodiment have an excellent cell-activating effect, they can be used for a cell-activating agent, a composition for cell activation, an external preparation for skin, a cosmetic preparation, and the like, which will be described later.

In the present specification, the mixture of the inositol derivatives of the first embodiment or the second embodiment may be referred to as the "present mixture of inositol derivatives."

### [Cell-activating agent]

In one embodiment, the present invention provides a cell-activating agent. The cell-activating agent of the present embodiment is characterized by including the mixture of inositol derivatives of the above-described first embodiment or second embodiment.

Since the present mixture of inositol derivatives has an excellent cell-activating effect, it can itself be used as a cell-activating agent. In addition, other components may be appropriately added to the mixture of inositol derivatives of the first embodiment or second embodiment so that the mixture of inositol derivatives may be used as a cell-activating agent. Examples of other components include pharmaceutically acceptable carriers to be described later.

The cell-activating agent of the present embodiment can be used by administering itself to a subject for the purpose of obtaining a cell-activating effect. In addition, the cell-activating agent of the present embodiment can be blended into pharmaceuticals or cosmetic preparations and used for the purpose of imparting a cell-activating function. Furthermore, it can be blended into a composition for cell activation to be described later and used.

The cell-activating agent of the present embodiment can be administered to a subject in the same manner as the composition for cell activation to be described later, and it is preferably administered transdermally.

### [Composition for cell activation]

In one embodiment, the present invention provides a composition for cell activation. The composition for cell activation of the present embodiment is characterized by including the mixture of inositol derivatives of the above-described first embodiment or second embodiment.

The composition for cell activation of the present embodiment can be produced by mixing the present mixture of inositol derivatives, and optionally a pharmaceutically acceptable carrier and other components and formulating them according to a general method (for example, a method described in the Japanese Pharmacopoeia).

In the present specification, the term "pharmaceutically acceptable carrier" refers to a carrier that does not inhibit physiological activity of an active ingredient and does not exhibit substantial toxicity with respect to its administration subject. The phrase "not exhibiting substantial toxicity" means that the component is not toxic to an administration subject if it is used at a general dosage. The pharmaceutically acceptable carrier is not particularly limited, and examples thereof include excipients, binders, disintegrants, lubricants, emulsifiers, stabilizers, diluents, solvents for injections, oily bases, moisturizers, touch sensation improvers, surfactants, polymers, thickening/gelling agents, solvents, propellants, antioxidants, reducing agents, oxidizing agents, chelating agents, acids, alkali, powders, inorganic salts, water, metal-containing compounds, unsaturated monomers, polyhydric alcohols, polymer additives, adjuvants, wetting agents, thickeners, tackifiers, oily raw materials, liquid matrices, fat-soluble substances, polymer carboxylate salts, and the like. Specific examples of these components include those described in PCT International Publication No. WO2016/076310. The pharmaceutically acceptable carrier may be used alone or in combination of two or more kinds thereof.

In addition, the other components are not particularly limited, but examples thereof include preservatives, antibacterial agents, ultraviolet absorbents, whitening agents, vitamins and derivatives thereof, antiphlogistics, anti-inflammatory agents, hair growth agents, blood circulation promoters, stimulants, hormones, anti-wrinkle agents, anti-aging agents, tightening agents, cooling sensation agents, warming sensation agents, wound healing promoters, irritation relieving agents, analgesics, cell activators, extracts of plants, animals, and microorganisms, antipruritic agents, exfoliating/dissolving agents, antiperspirants, refreshing agents, astringents, enzymes, nucleic acids, fragrances, colorants, coloring agents, dyes, pigments, anti-inflammatory analgesics, antifungals, antihistamines, hypnotic sedatives, tranquilizers, antihypertensives, antihypertensive diuretics, antibiotics, anesthetics, antibacterial substances, antiepileptic agents, coronary vasodilators, herbal medicines, antipruritic drugs, keratin softening and exfoliating agents, ultraviolet blockers, antiseptic disinfectants, antioxidant substances, pH adjusters, additives, metal soaps, and the like. Specific examples of these components include those described in PCT International Publication No. WO2016/076310. The other components may be used alone or in combination of two or more kinds thereof.

The composition for cell activation of the present embodiment may be a pharmaceutical composition, may be an external preparation for skin, or may be a cosmetic preparation.

### (Pharmaceutical composition)

In one embodiment, the present invention provides a pharmaceutical composition. The pharmaceutical composition of the present embodiment is characterized by including the mixture of inositol derivatives of the above-described first embodiment or second embodiment.

The pharmaceutical composition of the present embodiment may include a pharmaceutically acceptable carrier in addition to the present mixture of inositol derivatives. In the pharmaceutical composition of the present embodiment, the pharmaceutically acceptable carrier is not particularly limited, and carriers generally used for pharmaceuticals can be used in addition to the carriers exemplified above. For example, it is possible to use general raw materials described in the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (Edited by International Pharmaceutical Excipients Council Japan, Yakuji Nippo, Ltd., 2016), Handbook of Pharmaceutical Excipients, 7th edition (Pharmaceutical Press, 2012), and the like.

The pharmaceutically acceptable carrier may be used alone or in combination of two or more kinds thereof.

In addition, the pharmaceutical composition of the present embodiment may include other components in addition to the pharmaceutically acceptable carriers and the present mixture of inositol derivatives. The other components are not particularly limited, and general pharmaceutical additives can be used. In addition, active components other than the present mixture of inositol derivatives can also be used as the other components. As pharmaceutical additives and active ingredients as the other ingredients, in addition to those exemplified above, it is possible to use general raw materials described in, for example, the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (Edited by International Pharmaceutical Excipients Council Japan, Yakuji Nippo, Ltd., 2016), Handbook of Pharmaceutical Excipients, 7th edition (Pharmaceutical Press, 2012), and the like. The other components may be used alone or in combination of two or more kinds thereof.

A dosage form of the pharmaceutical composition of the present embodiment is not particularly limited, and it can be a dosage form generally used for pharmaceutical preparations. Examples thereof include orally administered dosage forms such as tablets, coated tablets, pills, powders, granules, capsules, solutions, suspensions, and emulsions; parenterally administered dosage forms such as injections, suppositories, and external preparations for the skin; and the like. The pharmaceutical composition in these dosage forms can be formulated according to a general method (for example, a method described in the Japanese Pharmacopoeia).

As the pharmaceutical composition of the present embodiment, an external preparation for the skin is preferable. More specific examples of external preparations for skin include dosage forms such as creams, lotions, packs, foams, skin cleansers, extracts, plasters, ointments, spirits, suspensions, tinctures, tapes, poultices, liniments, external aerosols, sprays, and gels.

The pharmaceutical composition of the present embodiment can contain a therapeutically effective amount of the present mixture of inositol derivatives. The term "therapeutically effective amount" refers to an amount of a drug effective for treating or preventing diseases of patients. The therapeutically effective amount may vary depending on a disease state, age, sex, body weight, and the like of an administration subject. In the pharmaceutical composition of the present embodiment, a therapeutically effective amount of the present mixture of inositol derivatives may be an amount at which the present mixture of inositol derivatives can exert a cell-activating effect. For example, a therapeutically effective amount of the present mixture of inositol derivatives in the pharmaceutical composition of the present embodiment may be 0.01 to 50 % by mass as the content of the inositol derivatives in the pharmaceutical composition, and it may be 0.01 to 30 % by mass, for example, it may be 0.01 to 20 % by mass for example, it may be 0.1 to 10 % by mass for example, it may be 0.1 to 5 % by mass for example, it may be 0.1 to 3 % by mass for example, it may be 0.3 to 2 % by mass for example, and it may be 0.6 to 1.5 % by mass, for example.

The content of the inositol derivatives in the pharmaceutical composition means a total content of all the inositol derivatives contained in the present mixture of inositol derivatives. The same applies to dosages to be described later.

A method for administering the pharmaceutical composition of the present embodiment is not particularly limited, and the pharmaceutical composition can be administered by a method generally used as a method for administering pharmaceuticals. For example, it may be administered orally as tablets, coated tablets, pills, powders, granules, capsules, solutions, suspensions, emulsions, and the like; it may be administered intravenously, intraarterially, intramuscularly, intradermally, subcutaneously, intraperitoneally, and the like as injections, infusion preparations, and the like alone, or as a mixture with common infusions such as a glucose solution and Ringer's solution; it may be administered rectally as suppositories; or it may be administered to skin as external preparations for the skin. In a preferable aspect, the pharmaceutical composition of the present embodiment is applied, affixed, or sprayed to an affected area as external preparations for the skin.

A dosage of the pharmaceutical composition of the present embodiment can be a therapeutically effective amount. The therapeutically effective amount may be appropriately determined according to symptoms, body weight, age, sex, and the like of a patient, a dosage form of the pharmaceutical composition, an administration method, and the like. For example, in the case of oral administration, a dosage of the pharmaceutical composition of the present embodiment may be 0.01 to 500 mg per unit of a dosage form as the present mixture of inositol derivatives; in the case of injections, the dosage may be 0.02 to 250 mg per unit of a dosage form as inositol derivatives; in the case of suppositories, the dosage may be 0.01 to 500 mg per unit of a dosage form as inositol derivatives; and the like. In the case of external preparations for the skin, for example, the dosage may be 0.15 to 500 mg per unit of a dosage form as inositol derivatives, may be 0.15 to 300 mg for example, may be 0.15 to 200 mg for example, or may be 0.2 to 100 mg, for example.

An administration interval of the pharmaceutical composition of the present embodiment may be appropriately determined according to symptoms, body weight, age, sex, and the like of a patient, a dosage form of the pharmaceutical composition, an administration method, and the like. For example, it may be once, about 2 to 3 times, or the like a day.

The pharmaceutical composition of the present embodiment can be used for promoting cell activation of skin cells in which a cell proliferative activity has been reduced, for example. In addition, for example, it can be used for promoting cell activation at sites where cells have been damaged due to wounds, burn injuries, cold injuries, inflammations, and the like. The pharmaceutical composition of the present embodiment is particularly effective for cell activation of human fibroblasts or human epidermal keratinocytes. In a preferable aspect, the pharmaceutical composition of the present embodiment is applied, as an external preparation for the skin, to an affected area where the cell proliferative activity has been reduced or an affected area where cell damage has occurred.

### (Cosmetic preparation)

In one embodiment, the present invention provides a cosmetic preparation. The cosmetic preparation of the present embodiment is characterized by including the mixture of inositol derivatives of the above-described first embodiment or second embodiment.

The cosmetic preparation of the present embodiment may include a pharmaceutically acceptable carrier in addition to the present mixture of inositol derivatives. In the cosmetic preparation of the present embodiment, the pharmaceutically acceptable carrier is not particularly limited, and carriers generally used for cosmetic preparations can be used in addition to the carriers exemplified above. For example, it is possible to use general raw materials described in the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The pharmaceutically acceptable carriers may be used alone or in combination of two or more kinds thereof.

The cosmetic preparation of the present embodiment may include other components in addition to the pharmaceutically acceptable carrier and the present mixture of inositol derivatives. The other components are not particularly limited, and general additives for cosmetic products can be used. In addition, active components other than the present mixture of inositol derivatives can also be used as the other components. As additives for cosmetic products and active ingredients as the other ingredients, in addition to those exemplified above, it is possible to use general raw materials described in, for example, the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. The other components may be used alone or in combination of two or more kinds thereof.

A form of the cosmetic preparation of the present embodiment is not particularly limited, and it can be a form generally used for a cosmetic preparation. Examples thereof include hair cosmetic preparations such as shampoos, hair conditioners, and hairdressing agents; basic cosmetic preparations such as facial cleansers, cleansing agents, skin toners, emulsions, lotions, creams, gels, sunscreens, packs, masks, and serums; makeup cosmetic preparations such as foundations, makeup primers, lipsticks, lip glosses, and blushers; body cosmetic preparations such as body cleansers, body powders, and deodorant cosmetics; and the like. These cosmetic preparations can be manufactured according to a general method. Among them, the cosmetic preparation of the present embodiment is preferably a cosmetic preparation in a form in which it is applied or attached to the skin as an external preparation for the skin. Preferable examples thereof include skin toners, emulsions, lotions, creams, gels, sunscreens, packs, masks, serums, foundations, makeup primers, and the like.

A dosage form of the cosmetic preparation of the present embodiment is not particularly limited, but examples thereof include emulsified types such as an oil-in-water (O/W) type, a water-in-oil (W/O) type, a W/O/W type, and an O/W/O type, emulsified polymer types, oily types, solid types, liquid types, kneaded types, stick types, volatile oil types, powder types, jelly types, gel types, paste types, cream types, sheet types, film types, mist types, spray types, multilayer types, foam types, flake types, and the like.

In the cosmetic preparation of the present embodiment, a content of the present mixture of inositol derivatives is not particularly limited, but it can be an effective amount for the present mixture of inositol derivatives to exert a cell-activating effect. For example, a content of the present mixture of inositol derivatives in the cosmetic preparation of the present embodiment may be 0.01 to 50 % by mass as a content of the inositol derivatives in the cosmetic preparation, and it may be 0.01 to 30 % by mass for example, it may be 0.01 to 20 % by mass for example, it may be 0.1 to 10 % by mass for example, it may be 0.1 to 5 % by mass for example, it may be 0.1 to 3 % by mass for example, it may be 0.3 to 2 % by mass for example, and it may be 0.6 to 1.5 % by mass for example.

In addition, the content of the inositol derivatives in the cosmetic preparation means a total content of all the inositol derivatives contained in the present mixture of inositol derivatives. The same applies to amounts used to be described later.

An amount of the cosmetic preparation of the present embodiment used is not particularly limited, but it can be an effective amount for the present mixture of inositol derivatives to exert a cell-activating effect. For example, an amount of the cosmetic preparation of the present embodiment used may be 0.15 to 500 mg per use as an amount of inositol derivatives, and it may be 0.15 to 300 mg for example, it may be 0.15 to 200 mg for example, and it may be 0.2 to 100 mg for example.

A use interval of the cosmetic preparation of the present embodiment is not particularly limited, but it can be, for example, once, about 2 to 3 times, or the like a day.

Since the cosmetic preparation of the present embodiment exhibits a cell-activating effect, it can be used for preventing and ameliorating wrinkles, spots, sagging, reduced elasticity, and the like of the skin. It can be expected that healthy skin would be maintained by using the cosmetic preparation of the present embodiment for daily skin care and makeup.

### [Method for producing present mixture of inositol derivatives]

The mixture of inositol derivatives of the first embodiment or the second embodiment can be produced as follows (hereinafter referred to as the "present production method"). The present production method includes a step of reacting inositol and oligosaccharides in the presence of a glycosyltransferase (hereinafter referred to as the "reaction step"). In the reaction step, the inositol and the oligosaccharides are preferably reacted at a mass ratio of 1:2 to 1:6.

### <Reaction step>

### (Inositol)

The inositol used in the reaction step may be any of the isomers listed in the above-described "[Mixture of inositol derivatives]," but it is preferably myo-inositol having a physiological activity. Inositol can be synthesized by a method of extraction from rice bran, a chemical synthesis method, a fermentation method, or the like. In addition, a commercially available product may also be used.

### (Oligosaccharides)

An oligosaccharide used in the reaction step is not particularly limited, and examples thereof include oligosaccharides containing mannitol, sorbitol, xylitol, erythritol, pentaerythritol, glucose, fructose, xylose, or the like as structural units. Examples of oligosaccharides include oligosaccharides having a molecular weight of about 500 to 3000. A degree of polymerization of the oligosaccharides is not particularly limited, but from the viewpoint of efficiency of generating the inositol derivative, it is possible to use, for example, oligosaccharides in which a content of oligosaccharides having a degree of polymerization of 7 or more is 85 % by mass or more, preferably 90 % by mass or more, and more preferably 98 % by mass or more.

Among the above examples, an oligosaccharide used in the reaction step is preferably an oligosaccharide containing glucose as a structural unit. Examples of oligosaccharides containing glucose as a structural unit include dextrin. Dextrin is a generic term applied to products obtained by reducing the molecular weight of starch by chemical or enzymatic methods. The dextrin is not particularly limited, but it is preferably a cyclodextrin from the viewpoint of efficiency of generating the inositol derivative. Cyclodextrins are cyclic oligosaccharides having a cyclic structure in which D-glucose units are bonded by α-1,4-glycosidic bonds. The cyclodextrin is not particularly limited, and examples thereof include α-cyclodextrin, β-cyclodextrin, γ-cyclodextrin, and the like. Among these, β-cyclodextrin is preferably used because it is industrially inexpensive and can be stably supplied.

Dextrin can be obtained by reducing the molecular weight of starch by chemical or enzymatic methods. In addition, cyclodextrin can be obtained by allowing cyclodextrin glucanotransferase to act on starch. A commercially available dextrin or cyclodextrin may be used.

### (Glycosyltransferase)

A glycosyltransferase used in the reaction step is not particularly limited, and it may be appropriately selected according to the type of oligosaccharide. For example, in a case where dextrin is used as an oligosaccharide, cyclodextrin glucanotransferase (hereinafter referred to as the "CGTase") can be used as the glycosyltransferase.

CGTase is an enzyme catalyzing a reaction in which an α-1,4-glucoside bond is formed to cyclize an α-1,4-glucan chain. CGTase acts on a substrate such as starch having an α-1,4-glucan chain to generate cyclodextrin. As CGTases, CGTases derived from bacteria such as the genus *Bacillus,* the genus *Brevibacterium,* the genus *Clostridium,* the genus *Corynebacterium,* the genus *Klebsiella,* the genus *Micrococcus,* the genus *Thermoanaerobacter,* and the genus *Thermoanaerobacterium* are known currently.

When inositol and dextrin are reacted in the presence of CGTase, CGTase acts on the dextrin, and transfer of glucose residues occurs with the inositol as an acceptor. As a result, an inositol derivative in which glucose and/or an oligosaccharide having glucose as a structural unit is bonded to inositol is generated.

CGTase is not particularly limited as long as it can generate the above-mentioned inositol derivatives from inositol and dextrin. CGTases derived from bacteria as described above may be used, or CGTases obtained by modifying these natural CGTases may be used. Examples of CGTase include CGTases disclosed in Japanese Unexamined Patent Application, First Publication No. S63-196596 and PCT International Publication No. WO96/33267, and the like, but examples are not limited thereto. A commercially available CGTase may be used.

### (Reaction of generating inositol derivatives)

In the reaction step, inositol and oligosaccharides are reacted at a mass ratio of 1:2 to 1:6 in the presence of the glycosyltransferase. A mass ratio between inositol and oligosaccharides is preferably 1:3 to 1:5 and is more preferably 1:3.5 to 1:4.5. The present mixture of inositol derivatives can be obtained by reacting inositol and oligosaccharides at the mass ratio as described above.

The reaction step can be performed using a method used in a general enzyme reaction without any particular limitation. Specifically, the inositol derivatives generation reaction can be performed by mixing inositol, oligosaccharides, and glycosyltransferase in a suitable solvent, and allowing a certain time to pass.

In the reaction step, regarding the solvent in which the substrate and glycosyltransferase are mixed, a buffer solution used for general enzyme reactions, or the like can be used without particular limitation. Examples of such buffer solutions include a citrate buffer solution, a phosphate buffer solution, a Tris-HCl buffer solution, a HEPES buffer solution, and the like, but examples are not limited thereto.

A concentration of inositol in a reaction solution is not particularly limited, but from the viewpoint of improving a batch production amount and avoiding a long reaction time, for example, the concentration thereof is 1 to 400 g/L, preferably 10 to 300 g/L, and more preferably 50 to 200 g/L.

An oligosaccharide concentration of a reaction solution may be set depending on the concentration of inositol in the reaction solution, and may be 2 to 6 times, preferably 3 to 5 times of a concentration (w/v) of inositol.

Regarding a method of adding the oligosaccharides, the oligosaccharides may be added initially all at once or may be added after the initial addition. In a case of adding an oligosaccharide latter, a total addition mass of initial and additional additions of oligosaccharides may be 2 to 6 times, preferably 3 to 5 times of an addition mass of inositol. In a case of adding an oligosaccharide later, a timing of addition is not particularly limited, and examples thereof include 4 hours, 8 hours, and 11 hours after the start of the reaction. An oligosaccharide is preferably added all at once.

A concentration of the glycosyltransferase in a reaction solution is not particularly limited, but for example, it is 0.01 to 100 g-SS/L, is preferably 0.05 to 50 g-SS/L, and is more preferably 0.1 to 10 g-SS/L.

Reaction conditions in the reaction step may be suitably set according to the type of glycosyltransferase. In a case of using a commercially available glycosyltransferase, reaction conditions can be set according to recommended conditions of the manufacturer. For example, in a case where CGTase is used as the glycosyltransferase, a reaction temperature is 20°C to 80°C, is preferably 30°C to 70°C, and is more preferably 40°C to 60°C. For example, a reaction pH is pH 3 to pH 9, is preferably pH 4 to pH 8, and is more preferably pH 5 to pH 7. In a case where the reaction temperature and pH are within these ranges, an enzyme activity of CGTase can be maintained in a high state.

A reaction time in the reaction step is not particularly limited, and it may be suitably set according to the type of glycosyltransferase, an amount of a reaction solution, and the like. From the viewpoint of efficiency of generating inositol derivatives and inhibiting production of residual unreacted products, for example, the reaction time is 5 to 300 hours, is preferably 30 to 70 hours, and is more preferably 40 to 60 hours. For example, the reaction may be performed until the disappearance of oligosaccharides can be confirmed by measuring the concentration of oligosaccharides in the reaction solution by LC-MS or the like.

By performing the reaction step as described above, inositol derivatives are generated in the reaction solution, and a solution containing the present mixture of inositol derivatives can be obtained.

### <Other steps>

The present production method may include other steps in addition to the reaction step. Examples of other steps include a step of removing a glycosyltransferase (a glycosyltransferase removal step), various steps that are generally used as means for purifying chemical substances (a purification step), and the like.

### (Glycosyltransferase removal step)

In the present production method, it is preferable to perform a glycosyltransferase removal step after the above-described reaction step. By performing the step of removing the glycosyltransferase, it is possible to obtain the present mixture of inositol derivatives which does not include a glycosyltransferase and impurities derived therefrom and which has a high degree of purification without altering the inositol derivatives,.

A method for removing a glycosyltransferase is not particularly limited, and examples thereof include a method using an ultrafiltration membrane. An ultrafiltration membrane is not particularly limited, but an ultrafiltration membrane that can be used for cross-flow ultrafiltration is preferable. In cross-flow ultrafiltration, a feed solution, which is subject to filtration, is allowed to flow in parallel to a membrane surface of the ultrafiltration membrane. Accordingly, solute molecules smaller than the pore size of the ultrafiltration membrane and some of the feed solution become a filtrate, and molecules larger than the pore size of the membrane are concentrated. Since various types of ultrafiltration membrane that can be used for cross-flow ultrafiltration are commercially available, an ultrafiltration membrane can be suitably selected among them and used. In the present specification, a "feed solution" refers to a liquid to be subjected to ultrafiltration, a "filtrate" refers to a liquid that has passed through an ultrafiltration membrane, and a "concentrate" refers to a liquid that has not passed through an ultrafiltration membrane.

A molecular weight cut-off of the ultrafiltration membrane can be within a range of 1000 to 100,000. In a case where the molecular weight cut-off is less than 1000, the filtration rate becomes low, causing deterioration in the efficiency in purification of inositol derivatives and productivity thereof. In a case where the molecular weight cut-off exceeds 100,000, there is a high probability of impurities derived from the glycosyltransferase being mixed into the filtrate. A molecular weight cut-off of the ultrafiltration membrane is preferably 1000 to 100,000, is more preferably 3000 to 20,000, and is even more preferably 4000 to 15000.

A method of ultrafiltration is not particularly limited, but it is preferably cross-flow ultrafiltration. By performing cross-flow ultrafiltration, adhesion of impurities to a surface of the ultrafiltration membrane can be reduced, and this can inhibit occurrence of clogging.

Temperature conditions at the time of ultrafiltration can be, for example, 0°C to 60°C, and it is preferably a temperature lower than the reaction temperature in the reaction step. In a case where the temperature is equal to or higher than the lower limit of the above temperature conditions, fluidity of the feed solution is maintained, and thereby filtration can be performed efficiently. In a case where the temperature is equal to or lower than the upper limit of these temperature conditions, overreaction due to the glycosyltransferase in the feed solution can be inhibited. Temperature conditions at the time of ultrafiltration are preferably 0°C to 50°C and more preferably 0°C to 40°C.

By performing ultrafiltration of a solution, which contains the present mixture of inositol derivatives obtained in the above-described reaction step, using an ultrafiltration membrane as described above, the glycosyltransferase and impurities derived therefrom remains in the concentrate, whereas the inositol derivative transfers to the filtrate. Accordingly, by recovering the filtrate, it is possible to obtain the present mixture of inositol derivatives with a high degree of purification which does not contain the glycosyltransferase and impurities derived therefrom.

It is preferable that a glycosyltransferase activity be not detected in an ultrafiltration filtrate obtained in the present step. Detection of the glycosyltransferase activity in the filtrate can be performed by, for example, collecting some of the filtrate, adding inositol (for example, myo-inositol) to a final concentration of 10 g/L, reacting the mixture at 50°C for 1 hour or longer, and thereafter, measuring a content of inositol in the reaction solution.

A content of inositol can be measured using, for example, high-performance liquid chromatography. In a case where there is no substantial difference in content of inositol between before and after the reaction, it can be determined that the glycosyltransferase activity has not been detected. The term "no substantial difference" means that, for example, a difference in content of inositol detected between before and after the reaction is about 5% or less with respect to a content of inositol detected before the reaction (100%). The difference in content of inositol is preferably 3% or less, is more preferably 2% or less, and is even more preferably 1 % or less.

### (Purification step)

In the present production method, a purification step may be performed after the reaction step. In a case of performing the glycosyltransferase removal step, the purification step is preferably performed after the glycosyltransferase removal step. In the purification step, a method generally used as a means for purifying chemical substances can be used without any particular limitation. Examples of purification means include treatment with an ion exchange resin or activated carbon, freeze-drying, spray-drying, and the like, but examples are not limited thereto. For example, a white powder of the present mixture of inositol derivatives can be obtained by freeze-drying or spray-drying a reaction solution obtained in the above-described reaction step or a filtrate obtained in the above-described glycosyltransferase removal step.

### (Other steps)

Examples of other steps that can be included in the present production method include, in addition to the above steps, a step of deactivating a glycosyltransferase, a step of analyzing the type of inositol derivative, a step of separating an inositol derivative having a specific number of monosaccharide units, and the like. These steps can be performed using a known method.

For example, a step of deactivating a glycosyltransferase (a glycosyltransferase deactivation step) can be performed by subjecting a reaction solution after the reaction step to a heat treatment (about 80°C to 120°C), or acid treatment or alkaline treatment. However, because inositol derivatives may be altered when performing the glycosyltransferase deactivation step, in the present production method, it is preferable not to perform the glycosyltransferase deactivation step.

### [Other embodiments]

In one embodiment, the present invention provides a method of promoting cell activation, the method including a step of administering, to a mammal, a mixture of inositol derivatives which contains an inositol derivative (A10) in which total sugars bonded to one inositol molecule is 10 or more in terms of monosaccharide units.

In one embodiment, the present invention provides a method of promoting cell activation, the method including a step of administering, to a mammal, a mixture of inositol derivatives which contains 5 % by mass or more of an inositol derivative (A7), in which total sugars bonded to one inositol molecule is 7 or more in terms of monosaccharide units, with respect to a total amount of inositol derivatives (100 % by mass).

In one embodiment, the present invention provides a mixture of inositol derivatives which contains an inositol derivative (A10) in which total sugars bonded to one inositol molecule is 10 or more in terms of monosaccharide units, for use in promoting cell activation.

In one embodiment, the present invention provides a mixture of inositol derivatives which contains 5 % by mass or more of an inositol derivative (A7), in which total sugars bonded to one inositol molecule is 7 or more in terms of monosaccharide units, with respect to a total amount of inositol derivatives (100 % by mass), for use in promoting cell activation.

In one embodiment, the present invention provides use of a mixture of inositol derivatives which contains an inositol derivative (A10) in which total sugars bonded to one inositol molecule is 10 or more in terms of monosaccharide units, for the manufacture of a cell-activating agent.

In one embodiment, the present invention provides use of a mixture of inositol derivatives which contains 5 % by mass or more of an inositol derivative (A7), in which total sugars bonded to one inositol molecule is 7 or more in terms of monosaccharide units, with respect to a total amount of inositol derivatives (100 % by mass), for the manufacture of a cell-activating agent.

In one embodiment, the present invention provides use of a mixture of inositol derivatives which contains an inositol derivative (A10) in which total sugars bonded to one inositol molecule is 10 or more in terms of monosaccharide units, for the manufacture of a composition for cell activation.

In one embodiment, the present invention provides use of a mixture of inositol derivatives which contains 5 % by mass or more of an inositol derivative (A7), in which total sugars bonded to one inositol molecule is 7 or more in terms of monosaccharide units, with respect to a total amount of inositol derivatives (100 % by mass), for the manufacture of a composition for cell activation,.

### [Examples]

Hereinafter, while the present invention will be described with reference to the following experimental examples, the present invention is not limited to the following experimental examples.

### [Experimental Example 1] Production of mixture of inositol derivatives (Example 1)

Under the condition shown in Table 3, using a 5 L culture tank (model number MD-300, manufactured by Marubishi Bioengineering Co., Ltd.), myo-inositol (manufactured by Tsuno Rice Fine Chemicals Co., Ltd.) and β-cyclodextrin (manufactured by ENSUIKO Sugar Refining Co., Ltd.) were reacted in the presence of CGTase (manufactured by Novozymes), and thereby inositol derivatives were generated. A "composition of reaction solution" in Table 3 indicates a final concentration.

**[Table 3]**

| | | |
|---|---|---|
| Composition of reaction solution | β-cyclodextrin | 459 g/L |
| | Myo-inositol | 116 g/L |
| | CGTase | 0.5-SS/L |
| | Anhydrous citric acid | 0.11 g/L |
| | Sodium citrate dihydrate | 1.37 g/L |
| | Water | |
| pH | 6.3 | |
| Temperature | 50°C | |
| Volume of reaction tank | 5 L | |
| Amount of reaction solution | 2.4 L | |
| Stirring rate | 200 rpm | |
| Reaction time | 48 hours | |

A diluted solution obtained by adding 3.0 L of water to 2.2 L of the reaction solution after the reaction was added to a stock solution tank of a membrane device in which a cross-flow ultrafiltration membrane (SIP-1013, Asahi Kasei Corporation) was placed. A circulation pump was operated to concentrate the stock solution to 1.1 L, and the filtrate was recovered. Thereafter, a step of adding 1 L of water to the stock solution tank and further recovering the filtrate was repeated 5 times. All the filtrates were mixed, and thereby 9.1 L of a recovered filtrate was obtained. Table 4 summarizes an amount of reaction solution, an amount of water added, and an amount of recovered filtrate in the present treatment. A "treatment time" indicates a time taken to obtain a recovered filtrate in which all the filtrates had been mixed.

**[Table 4]**

| | | |
|---|---|---|
| Amount of reaction solution | 2.2 L | |
| Amount of water initially added | 3 L | |
| Amount of water additionally added | 1 L × 5 | |
| Amount of recovered filtrate | 9.1 L | |
| Treatment time | 1.0 hour | |

The inositol derivatives were purified from the recovered filtrate, and thereby a mixture of the inositol derivatives was obtained. The obtained mixture of the inositol derivatives was subjected to HPLC analysis under the following conditions to examine a composition of the mixture of the inositol derivatives.

### <Analysis conditions>

Column: Shodex HILICpak VN-50 4D x 1
Eluent: CH₃CN:water = 60:40 (V:V)
Flow rate: 0.3 mL/min
Oven temperature: 40°C
Detection: R1 (differential refractive index)

The results are shown in Table 8. In Table 8, a proportion of each inositol derivative is based on a total amount (100 % by mass) of all inositol derivatives contained in the mixture.

### (Example 2)

Under the condition shown in Table 5, myo-inositol was reacted with β-cyclodextrin in the presence of CGTase, and thereby inositol derivatives were generated. A "composition of reaction solution" in Table 5 indicates a final concentration.

**[Table 5]**

| | | |
|---|---|---|
| Composition of reaction solution | β-cyclodextrin | 246 g/L |
| | Myo-inositol | 98 g/L |
| | CGTase | 1.0-SS/L |
| | Anhydrous citric acid | 0.11 g/L |
| | Sodium citrate dihydrate | 1.37 g/L |
| | Water | |
| pH | 6.3 | |
| Temperature | 50°C | |
| Volume of reaction tank | 1 L | |
| Amount of reaction solution | 0.51 L | |
| Stirring rate | 200 rpm | |
| Reaction time | 22.5 hours | |

The reaction solution after the reaction was ultrafiltered using a cross-flow ultrafiltration membrane (SIP-0013, Asahi Kasei Corporation), and the filtrate was recovered. The inositol derivatives were purified from the recovered filtrate, and further fractionated using activated carbon. 600 mL of the filtrate 10-fold diluted with ion exchange water was allowed to pass through an activated carbon column (a column in which a glass chromatograph tube with 40 mm diameter x 1000 mm was filled with activated carbon (manufactured by NACALAI TESQUE, INC., for column chromatography)), and thereafter, 400 mL of ion exchange water, 700 mL of 5% ethanol, 1000 mL of 10% ethanol, 1000 mL of 15% ethanol, 1400 mL of 20% ethanol, and 500 mL of 30% ethanol were allowed to pass through the activated carbon column sequentially. Thereafter, 50% ethanol was allowed to pass through the activated carbon column, the eluted fraction was recovered, and thereby a mixture of the inositol derivatives was obtained.

The obtained inositol derivatives were subjected to HPLC analysis in the same manner as in Example 1 to examine a composition of the mixture of the inositol derivatives. The results are shown in Table 8.

### (Example 3)

Under the condition shown in Table 6, myo-inositol was reacted with β-cyclodextrin in the presence of CGTase, and thereby inositol derivatives were generated. A "composition of reaction solution" in Table 6 indicates a final concentration.

**[Table 6]**

| | | |
|---|---|---|
| Composition of reaction solution | β-cyclodextrin | 258 g/L |
| | Myo-inositol | 110 g/L |
| | CGTase | 1.0-SS/L |
| | Anhydrous citric acid | 0.11 g/L |
| | Sodium citrate dihydrate | 1.37 g/L |
| | Water | |
| pH | 6.3 | |
| Temperature | 50°C | |
| Volume of reaction tank | 5 L | |
| Amount of reaction solution | 3.2 L | |
| Stirring rate | 250 rpm | |
| Reaction time [0131] | 24 hours | |

The reaction solution after the reaction was ultrafiltered using a cross-flow ultrafiltration membrane (SIP-0013, Asahi Kasei Corporation), and the filtrate was recovered. The inositol derivatives were purified from the recovered filtrate, and further fractionated using activated carbon. 1460 mL of the filtrate was allowed to pass through an activated carbon column (a column in which a glass chromatograph tube with 40 mm diameter x 1000 mm was filled with activated carbon (manufactured by NACALAI TESQUE, INC., for column chromatography)), and thereafter, 2070 mL of ion exchange water and 4140 mL of 10% ethanol were allowed to pass through the activated carbon column sequentially. The eluted fraction was recovered, and thereby a mixture of the inositol derivatives was obtained.

The obtained inositol derivatives were subjected to HPLC analysis in the same manner as in Example 1 to examine a composition of the mixture of the inositol derivatives. The results are shown in Table 8.

### (Example 4)

In the same manner as in Example 3, and under the condition shown in Table 6, myo-inositol was reacted with β-cyclodextrin in the presence of CGTase, and thereby inositol derivatives were generated.

The reaction solution after the reaction was ultrafiltered using a cross-flow ultrafiltration membrane (SIP-0013, Asahi Kasei Corporation), and the filtrate was recovered. The inositol derivatives were purified from the recovered filtrate, and further fractionated using activated carbon. 1460 mL of the filtrate was allowed to pass through an activated carbon column (a column in which a glass chromatograph tube with 40 mm diameter x 1000 mm was filled with activated carbon (manufactured by NACALAI TESQUE, INC., for column chromatography)), and thereafter, 2070 mL of ion exchange water was allowed to pass through the activated carbon column. The eluted fraction was recovered, and thereby a mixture of the inositol derivatives was obtained.

The obtained inositol derivatives were subjected to HPLC analysis in the same manner as in Example 1 to examine a composition of the mixture of the inositol derivatives. The results are shown in Table 8.

### (Comparative Example 1)

Under the condition shown in Table 7, myo-inositol was reacted with β-cyclodextrin in the presence of CGTase, and thereby inositol derivatives were generated. A "composition of reaction solution" in Table 7 indicates a final concentration.

**[Table 7]**

| | | |
|---|---|---|
| Composition of reaction solution | β-cyclodextrin | 224 g/L |
| | Myo-inositol | 73 g/L |
| | CGTase | 0.5-SS/L |
| | Anhydrous citric acid | 0.11 g/L |
| | Sodium citrate dihydrate | 1.37 g/L |
| | Water | |
| pH | 6.3 | |
| Temperature | 50°C | |
| Volume of reaction tank | 1 L | |
| Amount of reaction solution | 0.56 L | |
| Stirring rate | 200 rpm | |
| Reaction time | 22.5 hours | |

The reaction solution after the reaction was ultrafiltered using a cross-flow ultrafiltration membrane (SIP-0013, Asahi Kasei Corporation), and the filtrate was recovered. The inositol derivatives were purified from the recovered filtrate, and further fractionated using activated carbon. 611 mL of the filtrate 10-fold diluted with ion exchange water was allowed to pass through an activated carbon column (a column in which a glass chromatograph tube with 40 mm diameter x 1000 mm was filled with activated carbon (manufactured by NACALAI TESQUE, INC., for column chromatography)), and thereafter, 400 mL of ion exchange water, 780 mL of 10% ethanol, and 780 mL of 20% ethanol were allowed to pass through the activated carbon column sequentially. Thereafter, 780mL of 30% ethanol was allowed to pass through the activated carbon column, the eluted fraction was recovered, and thereby a mixture of the inositol derivatives was obtained.

The obtained inositol derivatives were subjected to HPLC analysis in the same manner as in Example 1 to examine a composition of the mixture of the inositol derivatives. The results are shown in Table 8.

**[Table 8]**

| Number of monosaccharide units | Example 1 | Example 2 | Example 3 | Example 4 | Comparative Example 1 |
|---|---|---|---|---|---|
| 1 | 9 | 0 | 8 | 3 | 1 |
| 2 | 12 | 5 | 20 | 13 | 20 |
| 3 | 12 | 5 | 20 | 16 | 30 |
| 4 | 12 | 14 | 17 | 16 | 24 |
| 5 | 11 | 22 | 13 | 13 | 14 |
| 6 | 9 | 21 | 9 | 11 | 7 |
| 7 | 8 | 15 | 6 | 8 | 3 |
| 8 | 6 | 9 | 4 | 7 | 1 |
| 9 | 5 | 4 | 2 | 9 | 0 |
| 10 | 4 | 2 | 0 | 0 | 0 |
| 11 | 4 | 1 | 0 | 2 | 0 |
| 12 or more | 8 | 2 | 1 | 2 | 0 |

| | | | | | |
|---|---|---|---|---|---|
| (Unit: % by mass) | | | | | |

As shown in Table 8, the mixtures of inositol derivatives of Examples 1 to 4 contained more inositol derivatives having a larger number of monosaccharide units than the mixture of inositol derivatives of Comparative Example 1. Regarding inositol derivatives having 7 or more monosaccharide units, 35 % by mass thereof was contained in Example 1, 33 % by mass thereof was contained in Example 2, 13 % by mass thereof was contained in Example 3, and 28 % by mass thereof was contained in Example 4, whereas only 4 % by mass of the inositol derivatives having 7 or more monosaccharide units was contained in Comparative Example 1. In addition, in Example 1, a proportion of inositol derivatives having 9 or more monosaccharide units was 21 % by mass, and a proportion of an inositol derivative having 10 or more monosaccharide units was 16 % by mass, whereas in Comparative Example 1, the inositol derivatives having 9 or more monosaccharide units were 0 % by mass. In Example 1, 12 % by mass of inositol derivatives having 11 or more monosaccharide units and 8 % by mass of inositol derivatives having 12 or more monosaccharide units were contained. In Examples 2 to 4, the inositol derivatives having 10 or more monosaccharide units were also contained. In Example 2, the inositol derivatives having 10 or more monosaccharide units, the inositol derivatives having 11 or more monosaccharide units, and the inositol derivatives having 12 or more monosaccharide units were contained. Among Examples 1 to 4, Example 1 had the highest content of the inositol derivatives having 10 or more monosaccharide units.

Based on the above results, it was confirmed that a mixture of inositol derivatives containing the inositol derivatives having 10 or more monosaccharide units can be obtained by reacting inositol and dextrin by the methods shown in Examples 1 to 4. In addition, it was also confirmed that a mixture of inositol derivatives which has a high content of inositol derivatives having a large number of monosaccharide units (monosaccharide units of 7 or more) can be obtained.

### [Experimental Example 2] Cell activation test using normal human fibroblasts

As cells, cells obtained by treating normal human fibroblasts (RIKEN BioResource Research Center) with hydrogen peroxide were used. As a medium, Dulbecco's modified Eagle medium (DMEM) containing 10% fetal bovine serum was used.

The mixtures of inositol derivatives of Examples 1 to 4 and Comparative Example 1 were dissolved in purified water and added to the medium so that a final concentration became 10 mg/L, and thereby a medium was prepared (Examples 1 to 4 and Comparative Example 1). As a control, a medium in which purified water was added instead of the inositol derivatives was prepared (control). The cells were cultured in these media for 24 hours at 37°C in a 5% CO₂ atmosphere.

After 24 hours from the start of culture, the cells were recovered and washed with phosphate buffered saline. Thereafter, a cell proliferative activity was measured. The measurement of the cell proliferative activity was performed by a method using WST-8 (a living cell count measuring reagent SF, NACALAI TESQUE, INC.), which is a tetrazolium salt that generates water-soluble formazan. Thereafter, the number of cells was measured by a neutral red method, and a value representing the cell proliferative activity was divided by a value representing the number of cells to calculate the cell activation activity.

The results are shown in Table 9. In Table 9, the cell activation activity was expressed as a relative value when defining the cell activation activity of the control in which purified water was added as 1.00. In Examples 1 to 4, it was confirmed that the cell activation activity was improved as compared with that of Comparative Example 1. Among Examples 1 to 4, the cell activation activity in Example 1 was the highest, and the cell activation activity in Example 3 was the lowest. Based on these results, it was confirmed that the cell activation activity improves as a content of the inositol derivatives having 7 or more monosaccharide units or the inositol derivatives having 10 or more monosaccharide units increases.

**[Table 9]**

| | Control | Example 1 | Example 2 | Example 3 | Example 4 | Comparative Example 1 |
|---|---|---|---|---|---|---|
| Addition concentration (mg/L) | Purified water | 10 | 10 | 10 | 10 | 10 |
| Cell activation activity | 1.00 | 1.25 | 1.21 | 1.12 | 1.15 | 0.98 |

### [Experimental Example 3] Cell activation test using normal human epidermal keratinocytes

As cells, cells obtained by subjecting normal human epidermal keratinocytes (NHEK cells, KURABO) to ultraviolet light irradiation were used. The ultraviolet light irradiation was carried out by recovering cells cultured in a medium, washing them with phosphate buffered saline, and then irradiating them with 60 mJ/cm² of ultraviolet B wave in the presence of phosphate buffered saline. As a medium, Dulbecco's modified Eagle medium (DMEM) containing 10% fetal bovine serum was used.

The mixtures of inositol derivatives of Examples 1 to 4 and Comparative Example 1 were dissolved in purified water and added to the medium so that a final concentration became 10 mg/L, and thereby a medium was prepared (Examples 1 to 4 and Comparative Example 1). As a control, a medium in which purified water was added instead of the inositol derivatives was prepared (control). The cells were cultured in these media for 24 hours at 37°C in a 5% CO₂ atmosphere.

After 24 hours from the start of culture, the cell proliferative activity was measured in the same manner as in Experimental Example 2, and the cell activation activity was calculated.

The results are shown in Table 10. In Table 10, the cell activation activity was expressed as a relative value when defining the cell activation activity of the control in which purified water was added as 1.00. In Examples 1 to 4, it was confirmed that the cell activation activity was improved as compared with that of Comparative Example 1. Among Examples 1 to 4, the cell activation activity in Example 1 was the highest, and the cell activation activity in Example 3 was the lowest. Based on these results, it was recognized that, even in the case of normal human epidermal keratinocytes, the cell activation activity tends to improve as a content of the inositol derivatives having 7 or more monosaccharide units or the inositol derivatives having 10 or more monosaccharide units increases.

**[Table 10]**

| | Control | Example 1 | Example 2 | Example 3 | Example 4 | Comparative Example 1 |
|---|---|---|---|---|---|---|
| Addition concentration (mg/L) | Purified water | 10 | 10 | 10 | 10 | 10 |
| Cell activation activity | 1.00 | 1.27 | 1.17 | 1.15 | 1.23 | 1.01 |

### [Industrial Applicability]

According to the present invention, a mixture of inositol derivatives which has an improved cell-activating effect, and use applications thereof are provided.

## Claims

1. A mixture of inositol derivatives in which a sugar is bonded to inositol, the mixture of inositol derivatives comprising an inositol derivative (A10) in which total sugars bonded to one inositol molecule is 10 or more in terms of monosaccharide units.

2. The mixture of inositol derivatives according to claim 1, comprising 1 % by mass or more of the inositol derivative (A10) with respect to a total amount of inositol derivatives (100 % by mass).

3. The mixture of inositol derivatives according to claim 2, comprising 5 % by mass or more of the inositol derivative (A10) with respect to a total amount of inositol derivatives (100 % by mass).

4. The mixture of inositol derivatives according to any one of claims 1 to 3, comprising an inositol derivative in which total sugars bonded to one inositol molecule is 11 or more in terms of monosaccharide units.

5. The mixture of inositol derivatives according to claim 4, comprising an inositol derivative in which total sugars bonded to one inositol molecule is 12 or more in terms of monosaccharide units.

6. The mixture of inositol derivatives according to any one of claims 1 to 5, wherein the inositol derivative (A10) is at least one kind of inositol derivatives selected from the group consisting of the following (a) and (b):
(a) an inositol derivative in which one or more glucoses, and one or more oligosaccharides containing glucose as a structural unit are respectively bonded to inositol; and
(b) an inositol derivative in which one or more oligosaccharides containing glucose as a structural unit are bonded to inositol.

7. The mixture of inositol derivatives according to any one of claims 1 to 6, further comprising:
an inositol derivative (B10) in which total sugars bonded to one inositol molecule is less than 10 in terms of monosaccharide units,
wherein the inositol derivative (B10) is at least one kind of inositol derivatives selected from the group consisting of the following (a) to (c):
(a) an inositol derivative in which one or more glucoses, and one or more oligosaccharides containing glucose as a structural unit are respectively bonded to inositol;
(b) an inositol derivative in which one or more oligosaccharides containing glucose as a structural unit are bonded to inositol; and
(c) an inositol derivative in which one or more glucoses are bonded to inositol.

8. A mixture of inositol derivatives in which a sugar is bonded to inositol, the mixture of inositol derivatives comprising 5 % by mass or more of an inositol derivative (A7), in which total sugars bonded to one inositol molecule is 7 or more in terms of monosaccharide units, with respect to a total amount of inositol derivatives (100 % by mass).

9. The mixture of inositol derivatives according to claim 8, wherein the inositol derivative (A7) is at least one kind of inositol derivatives selected from the group consisting of the following (a) and (b):
(a) an inositol derivative in which one or more glucoses, and one or more oligosaccharides containing glucose as a structural unit are respectively bonded to inositol; and
(b) an inositol derivative in which one or more oligosaccharides containing glucose as a structural unit are bonded to inositol.

10. The mixture of inositol derivatives according to claim 8 or 9, further comprising:
an inositol derivative (B7) in which total sugars bonded to one inositol molecule is less than 7 in terms of monosaccharide units,
wherein the inositol derivative (B7) is at least one kind of inositol derivatives selected from the group consisting of the following (a) to (c):
(a) an inositol derivative in which one or more glucoses, and one or more oligosaccharides containing glucose as a structural unit are respectively bonded to inositol;
(b) an inositol derivative in which one or more oligosaccharides containing glucose as a structural unit are bonded to inositol; and
(c) an inositol derivative in which one or more glucoses are bonded to inositol.

11. The mixture of inositol derivatives according to any one of claims 1 to 10, wherein the inositol is myo-inositol.

12. The mixture of inositol derivatives according to any one of claims 1 to 11, which promotes cell activation.

13. A cell-activating agent comprising the mixture of inositol derivatives according to any one of claims 1 to 12.

14. A composition for cell activation, comprising the mixture of inositol derivatives according to any one of claims 1 to 12.

15. An external preparation for skin, comprising the mixture of inositol derivatives according to any one of claims 1 to 12.

16. A cosmetic preparation comprising the mixture of inositol derivatives according to any one of claims 1 to 12.
